# EUROPÄISCHE PATENTSCHRIFT

(11) **EP 1 086 113 B1**
(45) Veröffentlichungstag und Bekanntmachung des Hinweises auf die Patenterteilung: **11.02.2004**
(21) Anmeldenummer: 99927802.1
(22) Anmeldetag: 29.05.1999
(51) Int. Cl.: C07H 15/26, C07D 337/08, A61K 31/70, A61K 31/38

(54) **BENZOTHIEPIN-1,1-DIOXIDDERIVATE, VERFAHREN ZU DEREN HERSTELLUNG, DIESE VERBINDUNGEN ENTHALTENDE ARZNEIMITTEL UND DEREN VERWENDUNG**
BENZOTHIEPINE-1,1-DIOXIDE DERIVATIVES, METHOD FOR PRODUCING THEM, MEDICAMENTS CONTAINING THESE COMPOUNDS AND THEIR USE
DERIVES DE BENZOTHIEPINE-1,1-DIOXYDE, PROCEDES PERMETTANT DE LES PREPARER, MEDICAMENTS CONTENANT LESDITS COMPOSES ET LEUR UTILISATION

(30) Priorität: 10.06.1998 DE 19825804
(43) Veröffentlichungstag der Anmeldung: 28.03.2001
(73) Patentinhaber: Aventis Pharma Deutschland GmbH, 65929 Frankfurt am Main (DE)
(72) Erfinder: FRICK, Wendelin, D-65510 Hünstetten-Beuerbach (DE); ENHSEN, Alfons, D-64572 Büttelborn (DE); GLOMBIK, Heiner, D-65719 Hofheim (DE); HEUER, Hubert, D-55270 Schwabenheim (DE)
(86) Internationale Anmeldenummer: PCT/EP1999/003743
(87) Internationale Veröffentlichungsnummer: WO 1999/064409

(56) Entgegenhaltungen:
- WO-A-96/08484
- WO-A-97/33882

## Beschreibung

Die Erfindung betrifft substituierte Benzothiepin-1,1-dioxidderivate, deren physiologisch verträgliche Salze und physiologisch funktionelle Derivate.

Es sind bereits Benzothiepin-1,1-dioxidderivate sowie deren Verwendung zur Behandlung von Hyperlipidämie sowie Arteriosklerose und Hypercholesterinämie beschrieben worden [vgl. PCT Anmeldung Nr. PCT/US97/04076, Veröffentlichungs- Nr. WO 97/33882].

Der Erfindung lag die Aufgabe zugrunde, weitere Verbindungen zur Verfügung zu stellen, die eine therapeutisch verwertbare hypolipidämische Wirkung entfalten. insbesondere bestand die Aufgabe darin, neue Verbindungen zu finden, die gegenüber den im Stand der Technik beschriebenen Verbindungen, bereits bei einer niedrigeren Dosierung eine höhere fäkale Gallensäureauscheidung bewirken. Eine Dosierungsreduzierung des ED₂₀₀ Wertes um mindestens den Faktor 5 gegenüber den im Stand der Technik beschriebenen Verbindungen war besonders wünschenswert.

Die Erfindung betrifft daher Verbindungen der Formel I, 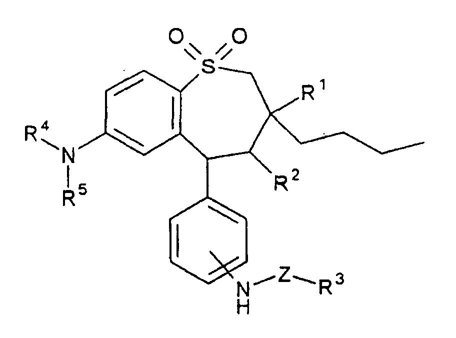 worin bedeuten
- R¹: Methyl, Ethyl, Propyl, Butyl;
- R²: H, OH, NH₂, NH-(C₁-C₆)-Alkyl;
- R³: Zuckerrest, Dizuckerrest, Trizuckerrest, Tetrazuckerrest, wobei der Zuckerrest, Dizuckerrest, Trizuckerrest oder Tetrazuckerrest gegebenenfalls ein oder mehrfach substituiert ist durch eine Zuckerschutzgruppe;
- R⁴: Methyl, Ethyl, Propyl, Butyl;
- R⁵: Methyl, Ethyl, Propyl, Butyl;
- Z: -(C=O)ₙ-C₀-C₁₆-Alkyl-, -(C=O)ₙ-C₀-C₁₆-Alkyl-NH-;
-(C=O)ₙ-C₀-C₁₆-Alkyl-O-, -(C=O)ₙ-C₁-C₁₆-Alkyl-(C=O)ₘ, eine kovalente Bindung;
- n: 0 oder 1;
- m: 0 oder 1;
sowie deren pharmazeutisch verträgliche Salze.

Bevorzugt sind Verbindungen der Formel 1, in denen ein oder mehrere Rest(e) die folgende Bedeutung hat bzw. haben:
- R¹: Ethyl, Propyl, Butyl;
- R²: H, OH, NH₂, NH-(C₁-C₆)-Alkyl;
- R²: H, OH, NH₂, NH-(C₁-C₆)-Alkyl;
- R³: Zuckerrest, Dizuckerrest, wobei der Zuckerrest oder Dizuckerrest, gegebenenfalls ein oder mehrfach substituiert ist durch eine Zucker-Schutzgruppe;
- R⁴: Methyl, Ethyl, Propyl, Butyl;
- R⁵: Methyl, Ethyl; Propyl, Butyl;
- Z: -(C=O)ₙ-C₀-C₁₆-Alkyl-, -(C=O)ₙ-C₀-C₁₆-Alkyl-NH-,
-(C=O)ₙ-C₀-C₁₆-Alkyl-O-,-(C=O)ₙ-C₁-C₁₆-Alkyl-(C=O)ₘ, eine kovalente Bindung;
- n: 0 oder 1;
- m: 0 oder 1;
sowie deren pharmazeutisch verträgliche Salze.

Besonders bevorzugt sind Verbindungen der Formel I, in denen ein oder mehrere Rest(e) die folgende Bedeutung hat bzw. haben:
- R¹: Ethyl, Butyl;
- R²: OH;
- R³: Zuckerrest, wobei der Zuckerrest gegebenenfalls ein oder mehrfach substituiert ist durch eine Zucker-Schutzgruppe;
- R⁴: Methyl;
- R⁵: Methyl;
- Z: -(C=O)-C₀-C₄-Alkyl, eine kovalente Bindung;
sowie deren pharmazeutisch verträgliche Salze.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter-, Sulfon- und Schwefelsäure sowie organischer Säuren, wie z.B. Essigsäure, Benzolsulfon-, Benzoe-, Zitronen-, Ethansulfon-, Fumar-, Glucon-, Glykol-, Isothion-, Milch-, Lactobion-, Malein-, Apfel-, Methansulfon-, Bernstein-, p-Toluolsulfon-, Wein- und Trifluoressigsäure. Für medizinische Zwecke wird in besonders bevorzugter Weise das Chlorsalz verwendet. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze).

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung, z.B. ein Ester, das bei Verabreichung an einen Säuger, wie z.B. den Menschen, in der Lage ist, (direkt oder indirekt) eine solche Verbindung oder einen aktiven Metaboliten hiervon zu bilden.

Ein weiterer Aspekt dieser Erfindung sind Prodrugs der erfindungsgemäßen Verbindungen. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Die erfindungsgemäßen Verbindungen eignen sich auch zur Prophylaxe bzw. zur Behandlung von Gallensteinen.

Nachfolgend beziehen sich alle Verweise auf "Verbindung(en) gemäß Formel (I)" auf Verbindung(en) der Formel (1) wie vorstehend beschrieben, sowie ihre Salze, Solvate und physiologisch funktionellen Derivate wie hierin beschrieben.

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,1 mg bis 100 mg (typischerweise von 0,1 mg und 50 mg) pro Tag pro Kilogramm Körpergewicht, z.B. 0,1-10 mg/kg/Tag. Tabletten oder Kapseln, können beispielsweise von 0,01 bis 100 mg, typischerweise von 0,02 bis 50 mg enthalten. Im Falle pharmazeutisch verträglicher Salze beziehen sich die vorgenannten Gewichtsangaben auf das Gewicht des vom Salz abgeleiteten Benzothiepin-Ions. Zur Prophylaxe oder Therapie der oben genannten Zustände können die Verbindungen gemäß Formel (1) selbst als Verbindung verwendet werden, vorzugsweise liegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (1). Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale und perorale (z.B. sublinguale) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (1) abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinalacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver oder Granulate; als Lösung oder Suspension in einer wäßrigen oder nicht-wäßrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Gegenstand der Erfindung sind weiterhin sowohl Isomerengemische der Formel I, als auch die reinen Stereoisomere der Formel I, sowie Diastereomerengemische der Formel I als auch die reinen Diastereomere. Die Trennung der Gemische erfolgt insbesonders auf chromatographischem Weg.

Bevorzugt sind racemische als auch enantiomerenreine Verbindungen der Formel I mit folgender Struktur: 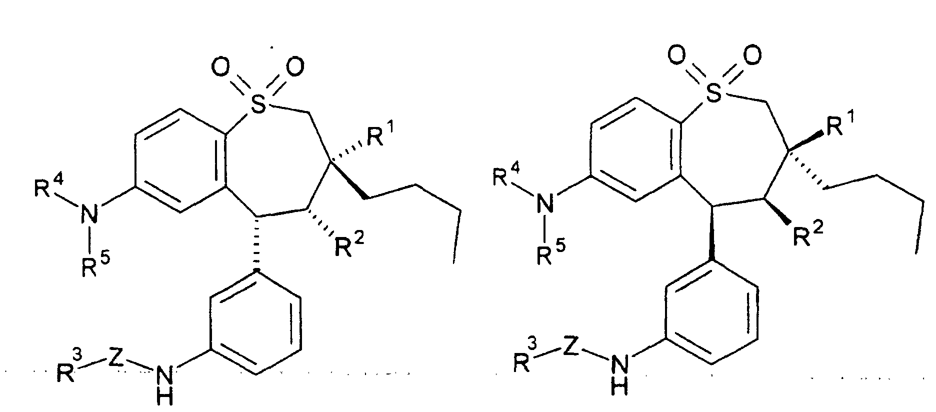 Unter Zuckerresten werden Verbindungen verstanden, die sich von Aldosen und Ketosen mit 3 bis 7 Kohlenstoffatomen ableiten, die der D- oder L-Reihe angehören können; dazu gehören auch Aminozucker, Zuckeralkohole oder Zuckersäuren. Beispielhaft seien genannt Glucose, Mannose, Fructose, Galaktose, Ribose, Erythrose, Glycerinaldehyd, Sedoheptulose, Glucosamin, Galaktosamin, Glucuronsäure, Galakturonsäure, Gluconsäure, Galaktonsäure, Mannonsäure, Glucamin, 3-Amino-1,2-propandiol, Glucarsäure und Galaktarsäure.

Bevorzugt sind die Zuckerreste:

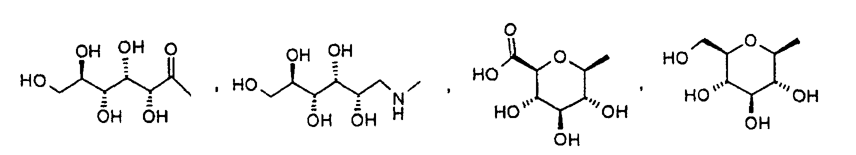

Besonders bevorzugt sind die Zuckerreste:

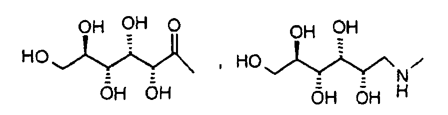

Mit Dizucker sind Saccharide gemeint, die aus zwei Zuckereinheiten bestehen.

Di-, Tri-, oder Tetrasaccharide entstehen durch acetalartige Bindung von 2 oder mehreren Zuckern. Die Bindungen können dabei in der α- oder β-Form auftreten. Beispielhaft seien genannt Laktose, Maltose und Cellobiose.

Wenn der Zucker substituiert ist, so erfolgt die Substitution bevorzugt am Wasserstoffatom einer OH-Gruppe des Zuckers.

Für die Hydroxygruppen der Zucker kommen im wesentlichen folgende Schutzgruppen in Frage: Benzyl-, Acetyl-, Benzoyl-, Pivaloyl-, Trityl-, tert.-Butyldimethylsilyl-, Benzyliden-, Cyclohexyliden- oder Isopropylidenschutzgruppen.

Die Erfindung betrifft weiterhin Verfahren zur Herstellung von Benzothiepin-1,1-dioxidderivate der Formel 1:

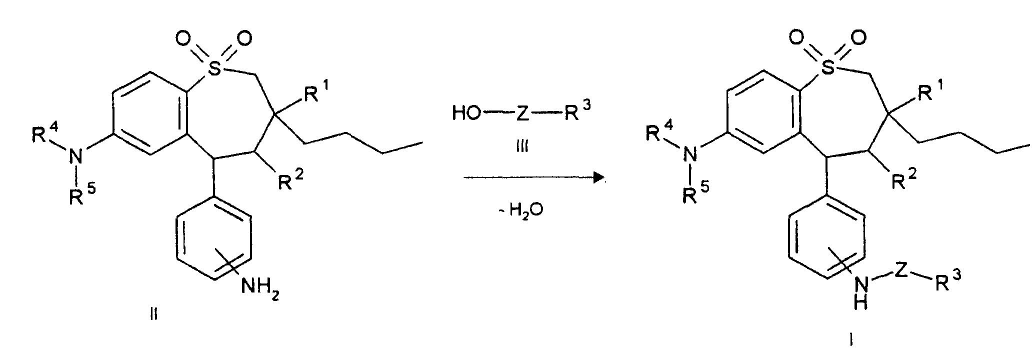

Verfahren zur Herstellung der Verbindungen der Forme I, dadurch gekennzeichnet, daß man ein Amin der Formel II, in der R', R², R⁴ und R⁵ die zu Formel I angegebenen Bedeutungen haben, mit einer Verbindung der Formel III, in der R³ und Z die zu Formel I angegebenen Bedeutungen haben, unter Wasserabspaltung zu einer Verbindung der Formel I umsetzt und die erhaltene Verbindung der Formel I gegebenenfalls in ein physiologisch verträgliche Salz oder ein physiologisch funktionelles Derivat überführt. Wenn es sich bei dem Rest R³ um einen Monozuckerest handelt, kann dieser Rest gegebenenfalls auch noch nach der Bindung an das Amin der Formel II stufenweise zum Dizuckerrest, Trizuckerrest oder Tetrazuckerrest verlängert werden.

Die Verbindungen der Formel I und deren pharmazeutisch verträgliche Salze und physiologisch funktionelle Derivate stellen ideale Arzneimittel zur Behandlung von Lipidstoffwechselstörungen, insbesondere von Hyperlipidämie dar. Die Verbindungen der Formel I eignen sich ebenfalls zur Beeinflussung des Serumcholesterinspiegels sowie zur Prävention und Behandlung arteriosklerotischer Erscheinungen. Die Verbindungen können gegebenenfalls auch in Kombination mit Statinen, wie z.B. Simvastatatin, Fluvastatin, Pravastatin, Cerivastatin, Lovastatin oder Atorvastin verabreicht werden. Folgende Befunde belegen die pharmakologische Wirksamkeit der erfindungsgemäßen Verbindungen.

Die biologische Prüfung der erfindungsgemäßen Verbindungen erfolgte durch Ermittlung der ED₂₀₀Ausscheidung. Diese Prüfung untersucht die Wirkung der erfindungsgemäßen Verbindungen auf den Gallensäurentransport im Ileum und die fäkale Ausscheidung von Gallensäuren bei der Ratte nach oraler Verabreichung zweimal täglich. Es wurden die Diastereomerengemische der Verbindungen geprüft.

Der Test wurde wie folgt durchgeführt:

### 1) Zubereitung der Test- und Referenzsubstanzen

Zur Formulierung einer wässerigen Lösung diente folgende Rezeptur: Die Substanzen wurden in adäquatem Volumen einer Solutol (=Polyethylenglykol 600 Hydroxystearat; BASF, Ludwigshafen,Deutschland; Chargennr. 1763) enthaltenden wässrigen Lösung gelöst, so daß eine Endkonzentration von 5% Solutol in der wässerigen Lösung vorliegt. Die Lösungen/Suspensionen wurden in einer Dosierung von 5ml/kg per os verabreicht.

### 2) Versuchsbedingungen

Männliche Wistar Ratten (Kastengrund, Hoechst AG, Gewichtsbereich 250-350g) wurden in Gruppen zu jeweils 6 Tieren und ab 10 Tagen vor Behandlungsbeginn (Tag 1) bei einem umgekehrten Tag/Nacht Rhythmus (4^{οο} 16^{οο}dunkel, 16ºº - 4ºº hell) gehalten und erhielten eine Standard Futtermischung (Altromin, Lage, Deutschland). Drei Tage vor Versuchsbeginn (Tag 0) wurden die Tiere in Gruppen mit jeweils 4 Tieren eingeteilt.

Einteilung der Tiere in Behandlungsgruppen:

| Nummer der Gruppe | Tiernr. / Analysennr. | Testsubstanz¹ | Dosis (mg/kg/d) |
|---|---|---|---|
| | | | |
| 1 | 1-4 | negative Kontrolle | Trägersubstanz |
| 2 | 5-8 | Testsubstanz Dosis 1 | 2 × 0,008 |
| 3 | 9-12 | Testsubstanz Dosis 2 | 2 × 0,02 |
| 4 | 13-16 | Testsubstanz Dosis 3 | 2 × 0,1 |
| 5 | 17-20 | Testsubstanz Dosis 4 | 2 × 0,5 |

| | | | |
|---|---|---|---|
| ¹ gelöst/suspendiert in 5% Solutol HS 15/0,4% Stärkeschleim | | | |

### 3) Versuchsablauf

Nach intravenöser oder subkutaner Verabreichung von 5 µCi ¹⁴C-Taurocholat pro Ratte (Tag 0)wurden die Träger- oder Testsububstanzen um 7^{oo}-8^{oo} und um 15^{oo} - 16^{oo} des folgenden Tages (Tag 1) gegeben (Behandlung für einen Tag).
Kotproben für die Analyse von ¹⁴C-Taurocholat wurden alle 24 Stunden direkt nach der Verabreichung der morgendlichen Dosis genommen. Die Fäzes wurden gewogen, bei -18°C gelagert und später in 100 ml Aqua demineralisata suspendiert und homogenisiert (Ultra Turrax, Janke & Kunkel, IKA-Werk). Aliquote Teile (0,5 g) wurden gewogen und auf Verbrennungshütchen (Combusto Cones, Canberra Packard) in einer Verbrennungsapparatur (Tri Carb® 307 combuster Canberra Packard GmbH, Frankfurt am Main, Deutschland) verbrannt. Das entstandene ¹⁴CO₂ wurde mit Carbo-Sorb® (Canberra Packard) absorbiert. Die folgenden ¹⁴C Radioaktivitätsmessungen wurden nach Zugabe des Szintillators (Perma-Fluor complete scintillation cocktail Nr. 6013187, Packard) zu den Proben mit Hilfe der Flüssigszintillationszählung (LSC) bestimmt. Die fäkale Ausscheidung von ¹⁴C-Taurocholsäure wurde als kumulative und/oder prozentuale Restradioaktivität berechnet (siehe unten).

### 4) Beobachtungen und Messungen

Die fäkale Ausscheidung von ¹⁴C-TCA wurde in verbrannten aliquoten Teilen der in 24 Stunden-Intervallen genommenen Kotproben bestimmt, als "kumulativer Prozentsatz" der verabreichten Aktivität berechnet und als % der Restaktivität (=verbleibende Aktivität, d.h. verabreichte Aktivität abzüglich der bereits ausgeschiedenen Aktivität) ausgedrückt. Für die Berechnung der Dosis-Wirkungs-Kurven wurde die Ausscheidung von ¹⁴C Taurocholsäure als Prozentanteil der entsprechenden Werte der Kontrollgruppe (behandelt mit Trägersubstanz) ausgedrückt. Die ED₂₀₀, d.h. die Dosis, die die fäkale Ausscheidung von ¹⁴C Taurocholsäure auf 200% der Kontrollgruppe steigert, wird durch Interpolation aus einer sigmoiden oder linearen Dosis-Wirkungs-Kurve berechnet. Die kalkulierte ED₂₀₀ entspricht einer Dosis, die die fäkale Ausscheidung von Gallensäuren verdoppelt.

### 5) Ergebnisse

Tabelle 1 zeigt Meßwerte der ED₂₀₀ Ausscheidung.

**Tabelle 1:**

| Verbindungen aus Beispiel (Diastereomerengemisch) | ED₂₀₀ Ausscheidung (mg/kg/d) p.o. |
|---|---|
| 1 | 0,009 |
| 2 | 0,008 |
| 3 | 0,04 |
| 5 | 0,03 |
| 6 | 0,04 |
| 7 | 0,04 |
| 8 | 0,007 |
| 9 | 0,007 |
| 10 | 0,04 |
| 3 (reine Struktur 11a) | 0,008 |
| Vergleichsbeispiele | |
| 1 | 0,8 |
| 2 | 1,0 |
| 3 | 0,9 |

### 6) Diskussion

Aus den Meßdaten ist abzulesen, daß die erfindungsgemäßen Verbindungen der Formel I gegenüber den im Stand der Technik beschriebenen Verbindungen eine um den Faktor 20 bis 100 bessere Wirkung aufweisen.

Die nachfolgenden Beispiele dienen zur näheren Erläuterung der Erfindung, ohne dieselbe auf in den Beispielen beschriebene Produkte und Ausführungsformen einzuschränken.

### Beispiel 1

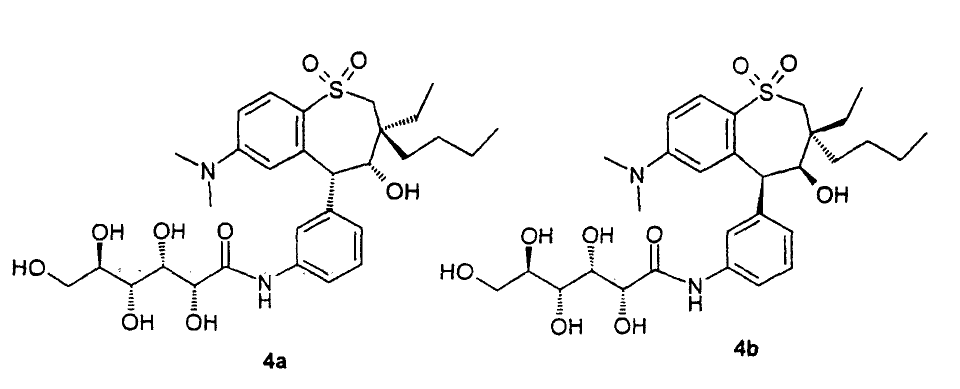

C₃₀H₄₄N₂O₉S (608.76). MS (M + H)⁺ = 609.3

### Beispiel 2

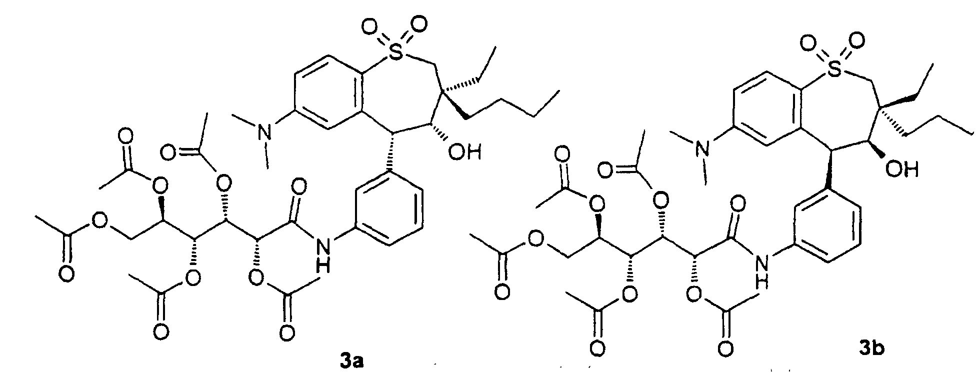

C₄₀H₅₄N₂O₁₄S (818.40), MS (M + H)⁺ = 819.3

### Beispiel 3

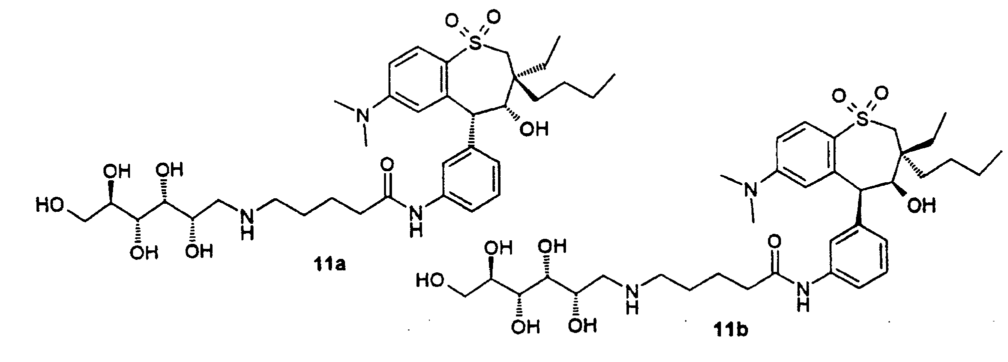

C₃₅H₅₅N₃O₉S (693.91). MS (M + H)⁺ = 694.4

### Beispiel 4

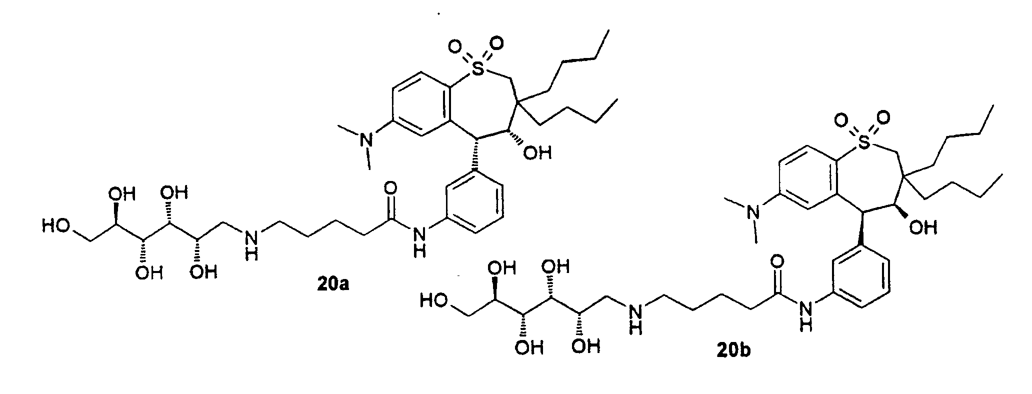

C₃₇H₅₉N₃O₉S (721.96). MS (M + H)⁺ = 722.3

### Beispiel 5

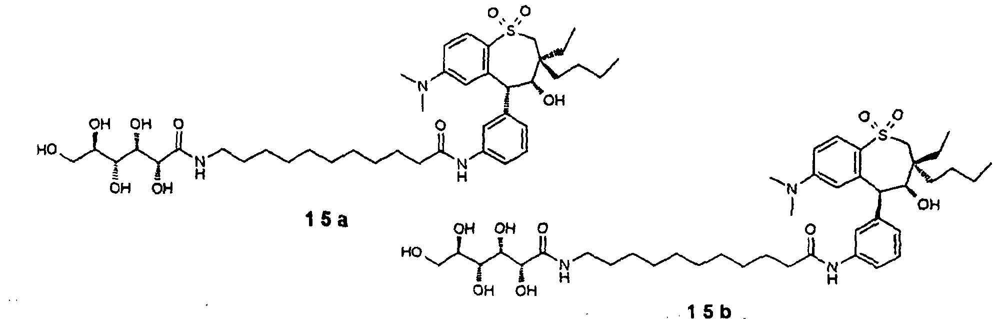

C₄₁H₆₅N₃O₁₀S (792.05). MS (M + H)⁺ = 792.5

### Beispiel 6

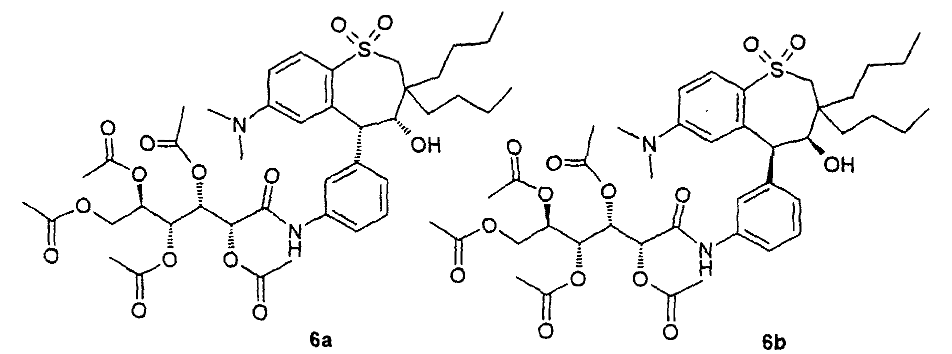

C₄₂H₅₈N₂O₁₄S (846.97), MS (M + H)⁺ = 847.4

### Beispiel 7

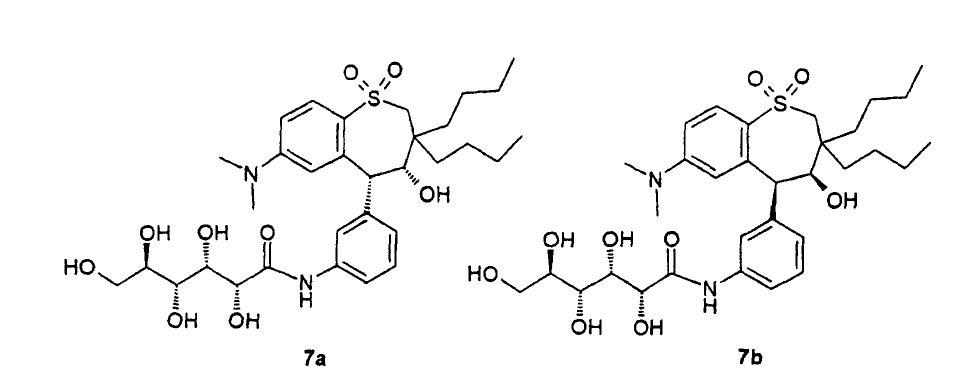

C₃₂H₄₈N₂O₉S (636.80). MS (M + H)⁺ = 637.4

### Beispiel 8

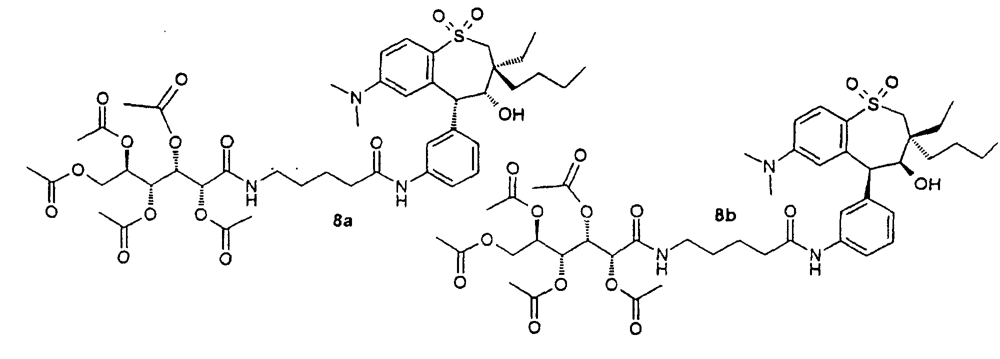

C₄₅H₆₃N₃O₁₅S (918.06). MS (M + H)⁺ = 918.6

### Beispiel 9

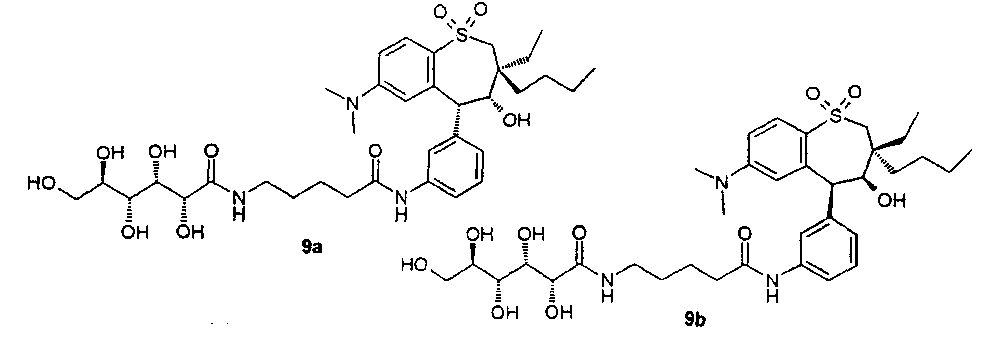

C₃₅H₅₃N₃O₁₀S (707.88). MS (M + H)⁺ = 708.4

### Beispiel 10

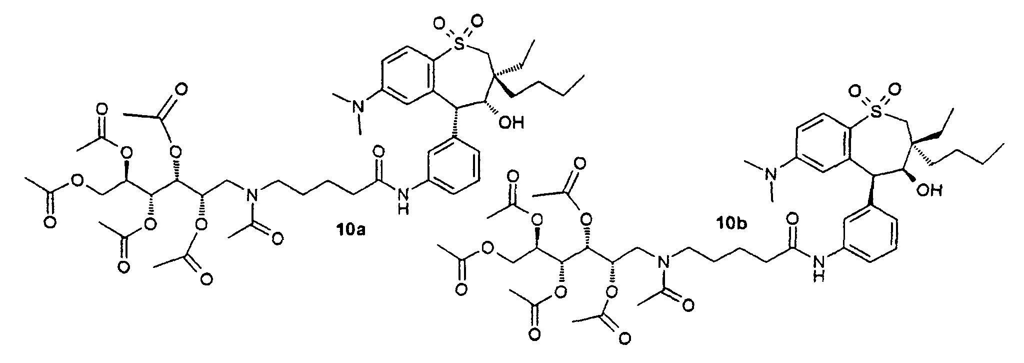

C₄₇H₆₇N₃O₁₅S (946.12). MS (M + H)⁺ = 946.5 Vergleichsbeispiele aus PCT/US97/04076:

### Vergleichsbeispiel 1

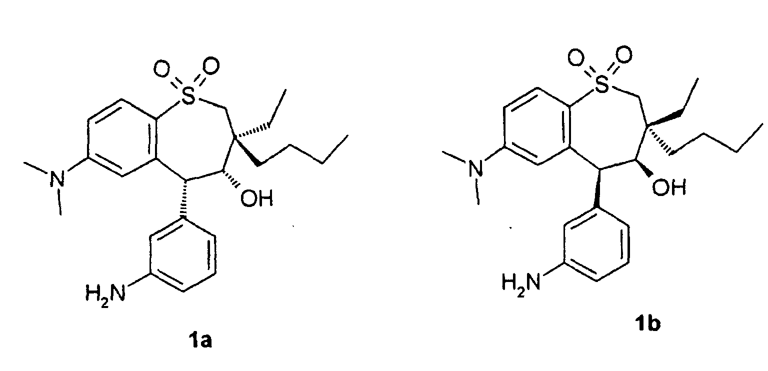

### Vergleichsbeispiel 2

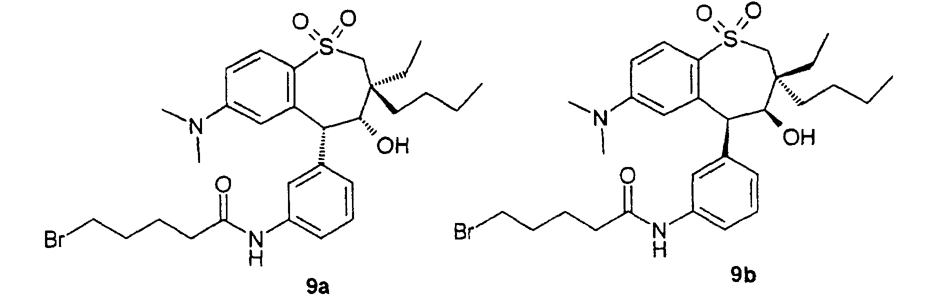

### Vergleichsbeispiel 3

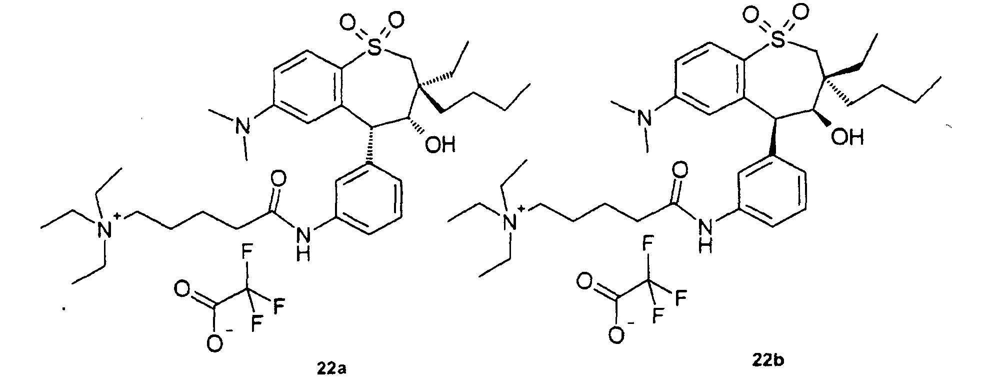

Die Beispiele bzw. Vergleichsbeispiele wurden wie folgt hergestellt (bei den Darstellungen wird nur die Synthese der a-Diastereomeren gezeigt):

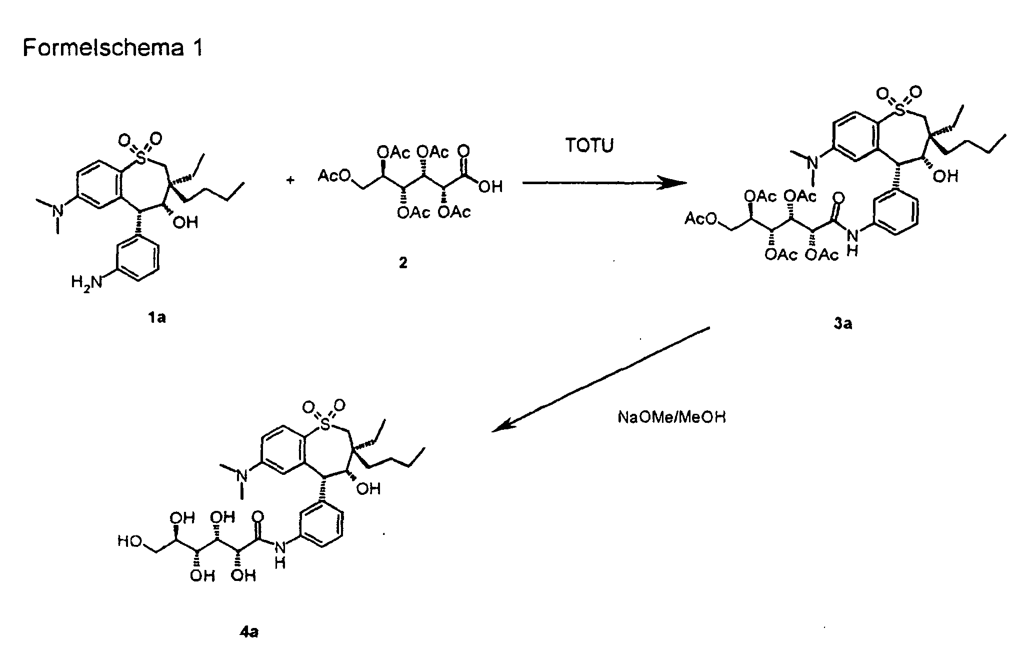

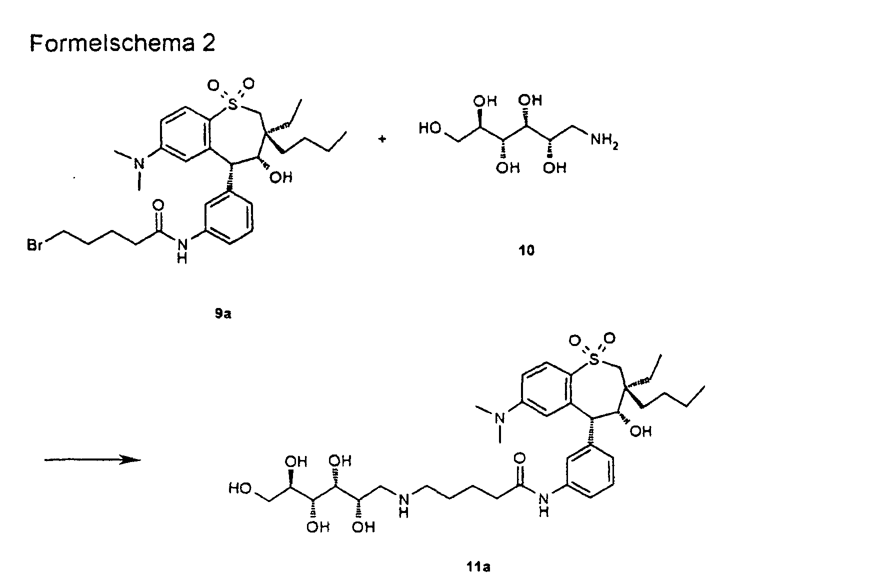

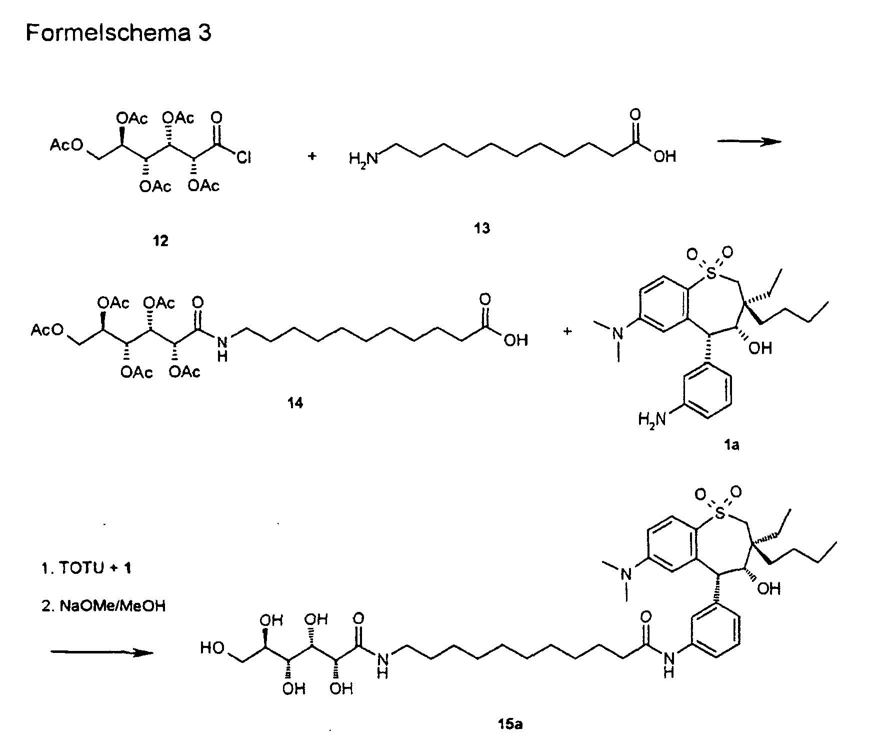

### Synthese von Verbindung 3 als Diastereomerengemisch:

300 mg (0.69mmol) **1a/b** (Herstellung analog PCT/ US 97/ 04076) und 700 mg (1.7 mmol) Penta- O- acetyl- D- gluconsäure (Org. Synth. Band 5, 887) werden in 10 ml DMF (Dimethylformamid) gelöst. Nacheinander werden dazu 700 mg (2.1 mmol) TOTU (Fluka), 250 mg (1.7 mmol) Oxim ( Hydroxyimino-cyanessigsäure- ethylester; Fluka) und 0.7 ml (5.5 mmol) NEM (4- Ethyl- morpholin) zugegeben. Nach einer Stunde bei Raumtemperatur wird mit 100 ml Ethylacetat verdünnt und dreimal mit Wasser gewaschen. Die organische Phase wird über MgSO₄ getrocknet, filtriert und eingeengt. Der Rückstand wird mittels Flashchromatographie (Ethylacetat/ n-Heptan 2:1) gereinigt und man erhält 502 mg (88%) **3a/b** als amorpher Feststoff. DC (Ethylacetat/ n-Heptan 2:1) R_{F}= 0.3. Das Produkt **3a/b** hat die gleiche Retention wie das Edukt **1a/b,** färbt allerdings mit 2 M Schwefelsäure unterschiedlich. C₄₀H₅₄N₂O₁₄S (818.40), MS (M + H)⁺ = 819.3.

### Synthese von Verbindung 4 als Diastereomerengemisch:

455 mg (0.55 mmol) **3a/b** werden in 20 ml Methanol gelöst. Nach Zugabe von 0.3 ml einer methanolischen 1 M Natriummethanolat- Lösung läßt man eine Stunde bei Raumtemperatur stehen. Dann wird mit methanolischer HCI- Lösung neutralisiert und eingeengt. Der Rückstand wird mit Flashchromatographie (Methylenchlorid/Methanol/ konz. Ammoniak 30/ 5/1) gereinigt und man erhält 280 mg (83%) **4a/b** als amorpher Feststoff. DC (Methylenchorid/ Methanol/ konz. Ammoniak 30/ 5/1). R_{f} = 0.2. C₃₀H₄₄N₂O₉S (608.76). MS (M + H)⁺ = 609.3.

### Synthese von Verbindung 11 als Diastereomerengemisch:

77 mg (0.013 mmol) **9a/b** (Herstellung analog PCT/ US 97/ 04076) werden in 4 ml DMF gelöst. Nach Zugabe von 150 mg (0.082 mmol) **10** (Glucamin, Fluka) wird zwei Stunden auf 80°C erwärmt. Danach wird mit 50 ml Ethylacetat verdünnt und dreimal mit Wasser gewaschen. Die organische Phase wird über MgSO₄ getrocknet, filtriert und eingeengt. Der Rückstand wird mittels Flashchromatografie (Methylenchlorid/ Methanol/ konz. Ammoniak 30/5/1) gereinigt und man erhält 55 mg (61 %) **11a/b** als amorphen Feststoff. DC (Methylenchlorid/ Methanol/ konz. Ammoniak 30/5/1). R_{f} = 0.3. C₃₅H₅₅N₃O₉S (693.91). MS (M + H)⁺ = 694.4.

### Synthese von Verbindung 14:

8.0 g (18.8 mmol) **12** (Penta-O-acetyl-D-gluconsäurechlorid; Org. Synth. Band 5, 887) werden zu einer Suspension von 8.0 g (40 mmol) **13** (Fluka) in 150 ml wasserfreiem DMF zugegeben. Diese Suspension wird 20 Stunden bei Raumtemperatur kräftig gerührt. Anschließend werden 500 ml Ethylacetat und 200 ml Wasser zugegeben. Die wässrige Phase wird nochmals mit 250 ml Ethylacetat extrahiert. Die vereinigte organische Phase wird dreimal mit Natriumchloridlösung gewaschen, über MgSO₄ getrocknet, filtriert und eingeengt. Ausbeute 9.5 g (86%) **14** als farbloses Öl. DC (Methylenchlorid/ Methanol/ konz. Ammoniak 3011013). R_{f}= 0.8. C₂₇H₄₃NO₁₃ (589.64). MS (M + H)⁺ = 590.4.

### Synthese von Verbindung 15 als Diastereomerengemisch:

200 mg (0.34 mmol) **14**, 78 mg (0.18 mmol) **1a/b**, 240 mg TOTU, 80 mg Oxim und 0.3 ml NEM werden in 4 ml DMF analog der Vorschrift für Verbindung **4** umgesetzt. Nach Flashchromatographie (Methylenchlorid/ Methanol/ konz. Ammoniak 30/5/1) erhält man 47 mg (33%, über zwei Stufen) **15a/b** als amorpher Feststoff. DC (Methylenchlorid/ Methanol/ konz. Ammoniak 30/5/1). R_{f}= 0.2. C₄₁H₆₅N₃O₁₀S (792.05). MS (M + H)⁺ = 792.5.

## Patentansprüche

1. Verbindungen der Formel I, 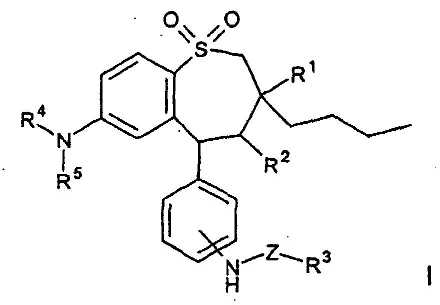 worin bedeuten
R¹ Methyl, Ethyl, Propyl, Butyl;
R² H, OH, NH₂, NH-(C₁-C₆)-Alkyl;
R³ Zuckerrest, Dizuckerrest, Trizuckerrest, Tetrazuckerrest,, wobei der Zuckerrest, Dizuckerrest, Trizuckerrest oder Tetrazuckerrest gegebenenfalls ein oder mehrfach substituiert ist durch eine Zucker-Schutzgruppe;
R⁴ Methyl, Ethyl, Propyl, Butyl;
R⁵ Methyl, Ethyl, Propyl, Butyl;
Z -(C=O)ₙ-C₀-C₁₆-Alkyl-, -(C=O)ₙ-C₀-C₁₆-Alkyl-NH-,
-(C=O)ₙ-C₀-C₁₆-Alkyl-O-, -(C=O)ₙ-C₁-C₁₆-Alkyl-(C=O)ₘ, eine kovalente Bindung;
n 0 oder 1;
m 0 oder 1;
sowie deren pharmazeutisch verträgliche Salze.

2. Verbindungen der Formel 1, gemäß Anspruch 1, **dadurch gekennzeichnet**, das einer oder mehrere der Reste die folgende Bedeutung hat bzw. haben:
R¹ Ethyl, Propyl, Butyl;
R² H, OH, NH₂, NH-(C₁-C₆)-Alkyl;
R³ Zuckerrest, Dizuckerrest, wobei der Zuckerrest oder Dizuckerrest, gegebenenfalls ein oder mehrfach substituiert ist durch eine Zucker-Schutzgruppe;
R⁴ Methyl, Ethyl, Propyl, Butyl;
R⁵ Methyl, Ethyl, Propyl, Butyl;
Z -(C=O)ₙ-C₀-C₁₆-Alkyl-, -(C=Oₙ-C₀-C₁₆-Alkyl-NH-,
-(C=O)ₙ-C₀-C₁₆-Alkyl-O-,-(C=O)ₙ,-C₁-C₁₆-Alkyl-(C=O)ₘ, eine kovalente Bindung;
n 0 oder 1;
m 0 oder 1;
sowie deren pharmazeutisch verträgliche Salze.

3. Verbindungen der Formel I, gemäß Anspruch 1 oder 2, **dadurch gekennzeichnet, daß** einer oder mehrere der Reste die folgende Bedeutung hat bzw. haben:
R¹ Ethyl, Butyl;
R² OH;
R³ Zuckerrest, wobei der Zuckerrest gegebenenfalls ein oder mehrfach substituiert ist durch eine Zucker-Schutzgruppe;
R⁴ Methyl;
R⁵ Methyl;
Z -(C=O)-C₀-C₄-Alkyl, eine kovalente Bindung;
sowie deren pharmazeutisch verträgliche Salze.

4. Verbindung der Formel I, gemäß einem oder mehreren der Ansprüche 1 bis 3, **dadurch gekennzeichnet, daß** die Verbindung die folgende Struktur besitzt 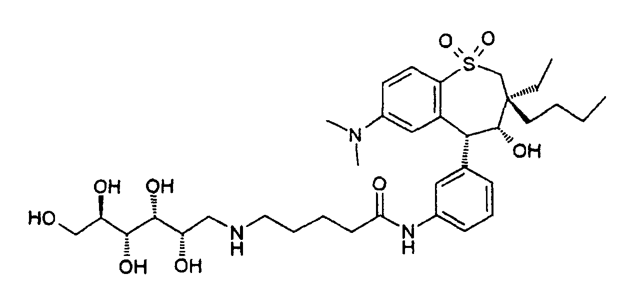 sowie deren pharmazeutisch verträgliche Salze.

5. Verbindung der Formel 1, gemäß einem oder mehreren der Ansprüche 1 bis 3, **dadurch gekennzeichnet, daß** die Verbindung die folgende Struktur besitzt 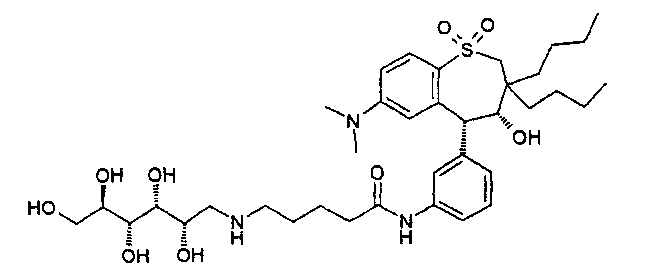 sowie deren pharmazeutisch verträgliche Salze.

6. Verfahren zur Herstellung von Verbindungen der Formel I, gemäß einem oder mehreren der Ansprüche 1 bis 5, **dadurch gekennzeichnet, daß** man nach folgendem Formelschema 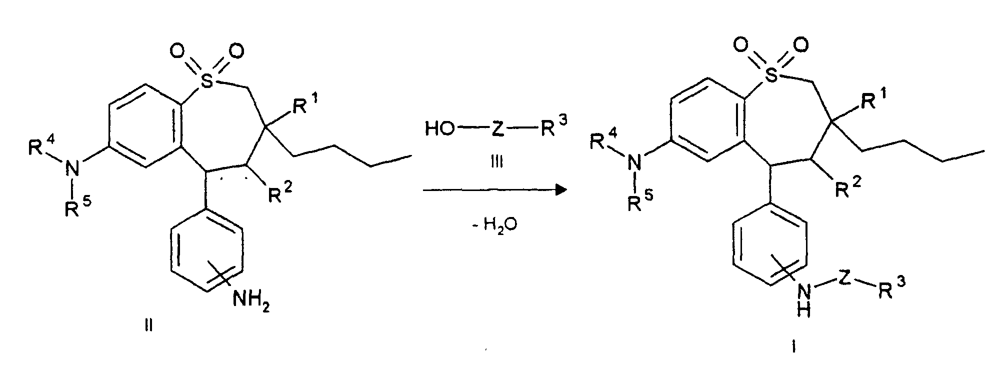 ein Amin der Formel II, in der R¹, R², R⁴ und R⁵ die zu Formel I angegebenen Bedeutungen haben, mit einer Verbindung der Formel III, in der R³ und Z die zu Formel I angegebenen Bedeutungen haben, unter Wasserabspaltung zu einer Verbindung der Formel I umsetzt und die erhaltene Verbindung der Formel I gegebenenfalls in ein physiologisch verträgliche Salz oder ein physiologisch funktionelles Derivat überführt.

7. Arzneimittel enthaltend eine oder mehrere der Verbindungen gemäß einem oder mehreren der Ansprüche 1 bis 5.

8. Arzneimittel enthaltend eine oder mehrere der Verbindungen gemäß einem oder mehreren der Ansprüche 1 bis 5 und ein oder mehrere Statine.

9. Verbindungen gemäß einem oder mehreren der Ansprüche 1 bis 5 zur Anwendung als Medikament zur Behandlung von Lipidstoffwechsetstörungen.

10. Verbindungen gemäß einem oder mehreren der Ansprüche 1 bis 5 zur Anwendung als Medikament zur Behandlung oder Prophylaxe von Gallensteinen.

11. Verfahren zur Herstellung eines Arzneimittels enthaltend eine oder mehrere der Verbindungen gemäß einem oder mehreren der Ansprüche 1 bis 5, **dadurch gekennzeichnet, daß** der Wirkstoff mit einem pharmazeutisch geeigneten Träger vermischt wird und diese Mischung in eine für die Verabreichung geeignete Form gebracht wird.

12. Verwendung der Verbindungen gemäß einem oder mehreren der Ansprüche 1 bis 5 zur Herstellung eines Medikaments zur Behandlung von Hyperlipidämie.

13. Verwendung der Verbindungen gemäß einem oder mehreren der Ansprüche 1 bis 5 zur Herstellung eines Medikaments zur Beeinflussung des Serumcholesterinspiegels.

14. Verwendung der Verbindungen gemäß einem oder mehreren der Ansprüche 1 bis 5 zur Herstellung eines Medikaments zur Prävention arteriosklerotischer Erscheinungen.

15. Verwendung der Verbindungen gemäß einem oder mehreren der Ansprüche 1 bis 5 zur Herstellung eines Medikaments zur Behandlung oder Prophylaxe von Gallensteinen.

## Claims

1. A compound of the formula I 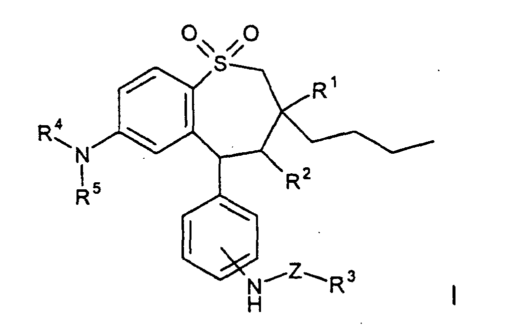 in which
R¹ is methyl, ethyl, propyl, butyl;
R² is H, OH, NH₂, NH-(C₁-C₆)-alkyl;
R³ is a sugar radical, a disugar radical, a trisugar radical, a tetrasugar radical, the sugar radical, disugar radical, trisugar radical or tetrasugar radical optionally being mono- or polysubstituted by a sugar protective group;
R⁴ is methyl, ethyl, propyl, butyl;
R⁵ is methyl, ethyl, propyl, butyl;
Z is -(C=O)ₙ-C₀-C₁₆-alkyl, -(C=O)ₙ-C₀-C₁₆-alkyl-NH-, -(C=O)ₙ-C₀-C₁₆-alkyl-O-, -(C=O)ₙ-C₁-C₁₆-alkyl-(C=O)ₘ, a covalent bond;
n is 0 or 1;
m is 0 or 1;
or its pharmaceutically tolerable salts.

2. A compound of the formula I as claimed in claim 1, wherein one or more of the radicals has or have the following meaning:
R¹ is ethyl, propyl, butyl;
R² is H, OH, NH₂, NH-(C₁-C₆)-alkyl;
R³ is a sugar radical, disugar radical, the sugar radical or disugar radical optionally being mono- or polysubstituted by a sugar protective group;
R⁴ is methyl, ethyl, propyl, butyl;
R⁵ is methyl, ethyl, propyl, butyl;
Z is -(C=O)ₙ-C₀-C₁₆-alkyl-, -(C=O)ₙ-C₀-C₁₆-alkyl-NH-, -(C=O)ₙ-C₀-C₁₆-alkyl-O-, -(C=O)ₙ-C₁-C₁₆-alkyl-(C=O)ₘ, a covalent bond;
n is 0 or 1;
m is 0 or 1;
or its pharmaceutically tolerable salts.

3. A compound of the formula I as claimed in claim 1 or 2, wherein one or more of the radicals has or have the following meaning:
R¹ is ethyl, butyl;
R² is OH;
R³ is a sugar radical, the sugar radical optionally being mono- or polysubstituted by a sugar protective group;
R⁴ is methyl;
R⁵ is methyl;
Z is -(C=O)-C₀-C₄-alkyl, a covalent bond;
or its pharmaceutically tolerable salts.

4. A compound of the formula I as claimed in one or more of claims 1 to 3, wherein the compound has the following structure 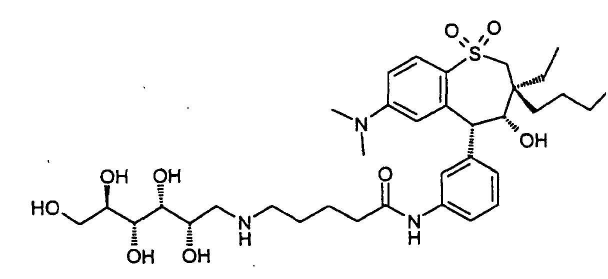 or its pharmaceutically tolerable salts.

5. A compound of the formula I as claimed in one or more of claims 1 to 3, wherein the compound has the following structure 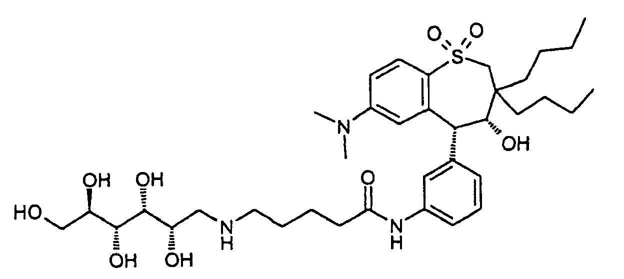 or its pharmaceutically tolerable salts.

6. A process for the preparation of compounds of the formula I as claimed in one or more of claims 1 to 5, which comprises reacting, according to the following equation, 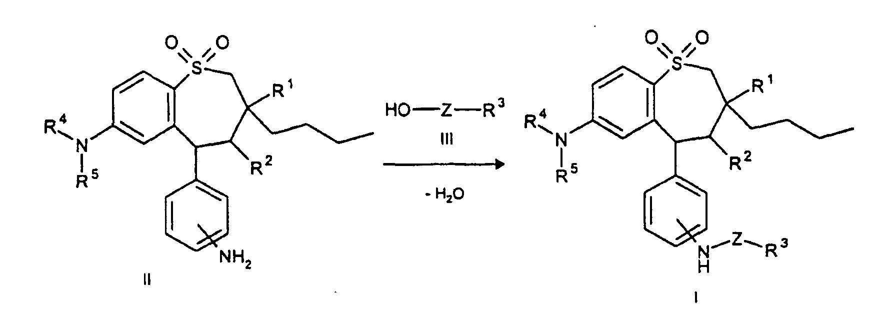 an amine of the formula II, in which R¹, R², R⁴ and R⁵ have the meanings indicated for formula I, with a compound of the formula III, in which R³ and Z have the meanings indicated for formula I, with elimination of water to give a compound of the formula I and optionally converting the compound of the formula I obtained into a physiologically tolerable salt or a physiologically functional derivative.

7. A pharmaceutical comprising one or more of the compounds as claimed in one or more of claims 1 to 5.

8. A pharmaceutical comprising one or more of the compounds as claimed in one or more of claims 1 to 5 and one or more statins.

9. A compound as claimed in one or more of claims 1 to 5 for use as a medicament for the treatment of lipid metabolism disorders.

10. A compound as claimed in one or more of claims 1 to 5 for use as a medicament for the treatment or prophylaxis of gallstones.

11. A process for the production of a pharmaceutical comprising one or more of the compounds as claimed in one or more of claims 1 to 5, which comprises mixing the active compound with a pharmaceutically suitable excipient and bringing this mixture into a form suitable for administration.

12. The use of the compounds as claimed in one or more of claims 1 to 5 for the production of a medicament for the treatment of hyperlipidemia.

13. The use of the compounds as claimed in one or more of claims 1 to 5 for the production of a medicament for influencing the serum cholesterol level.

14. The use of the compounds as claimed in one or more of claims 1 to 5 for the production of a medicament for the prevention of arteriosclerotic symptoms.

15. The use of the compounds as claimed in one or more of claims 1 to 5 for the production of a medicament for the treatment or prophylaxis of gallstones.

## Revendications

1. Composés de formule I, 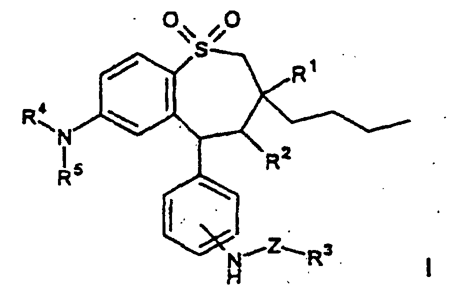 dans laquelle
R¹ représente un groupe méthyle, éthyle, propyle, butyle ;
R² représente un atome d'hydrogène, des groupes OH, NH₂, NH-(alkyle en C₁-C₆) ;
R³ représente un reste sucre, un reste disucre, un reste trisucre, un reste tétrasucre, le reste sucre, le reste disucre, le reste trisucre, le reste tétrasucre, éventuellement substitué une ou plusieurs fois par un groupe protecteur de sucre ;
R⁴ représente un groupe méthyle, éthyle, propyle, butyle ;
R⁵ représente un groupe méthyle, éthyle, propyle, butyle ;
Z représente -(C=O) ₙ-alkyle en C₀-C₁₆, -(C=O)ₙ-(alkyl en C₀-C₁₆)-NH-, -(C=O) ₙ-(alkyl en C₀-C₁₆) -O-, -(C=O)ₙ-(alkyl en C₀-C₁₆) -(C=O)ₘ-, une liaison covalente ;
n vaut 0 ou 1 ;
m vaut 0 ou 1 ;
ainsi que leurs sels tolérés du point de vue pharmaceutique.

2. Composés de formule I, selon la revendication 1, **caractérisés en ce que** un ou plusieurs restes possède(nt) les significations suivantes :
R¹ représente un groupe éthyle, propyle, butyle ;
R² représente un atome d'hydrogène, un groupe OH, NH₂, NH-(alkyle en C₁-C₆) ;
R³ représente un reste sucre, un reste disucre, le reste sucre ou le reste disucre sont éventuellement substitués une ou plusieurs fois par un groupe protecteur de sucre ;
R⁴ représente un groupe méthyle, éthyle, propyle, butyle ;
R⁵ représente un groupe méthyle, éthyle, propyle, butyle ;
Z représente -(C=O)ₙ-alkyle en C₀-C₁₆, -(C=O)ₙ-(alkyl en C₀-C₁₆) -NH-, -(C=O)ₙ-(alkyl en C₀-C₁₆) -O-. -(C=O)ₙ-(alkyl en C₀-C₁₆)-(C=O)ₘ-, une liaison covalente ;
n vaut 0 ou 1 ;
m vaut 0 ou 1 ;
ainsi que leurs sels tolérés du point de vue pharmaceutique.

3. Composés de formule I, selon les revendications 1 ou 2, **caractérisés en ce qu'**un ou plusieurs restes possède(nt) les significations suivantes :
R¹ représente un groupe éthyle, butyle ;
R² représente un groupe OH ;
R³ représente un reste sucre, le reste sucre étant éventuellement substitué une ou plusieurs fois par un groupe protecteur de sucre ;
R⁴ représente un groupe méthyle ;
R⁵ représente un groupe méthyle ;
Z représente -(C=O) -alkyle en C₀-C₄, une liaison covalente ;
ainsi que leurs sels tolérés du point de vue pharmaceutique.

4. Composé de formule I, selon une ou plusieurs des revendications 1 à 3, **caractérisé en ce que** le composé possède la structure suivante : 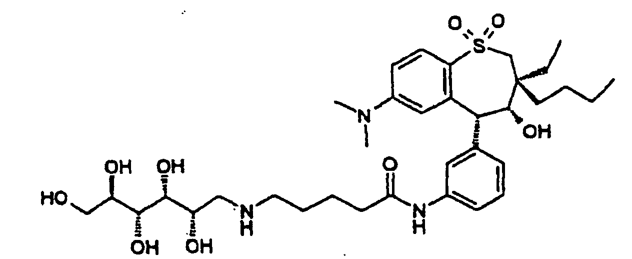 ainsi que ses sels tolérés du point de vue pharmaceutique.

5. Composé de formule I, selon une ou plusieurs des revendications 1 à 3, **caractérisé en ce que** le composé possède la structure suivante : 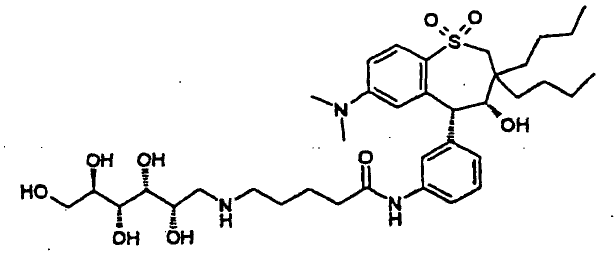 ainsi que ses sels tolérés du point de vue pharmaceutique.

6. Procédé pour la préparation de composés de formule I selon une ou plusieurs des revendications 1 à 5, **caractérisé en ce qu'**on transforme selon le schéma suivant 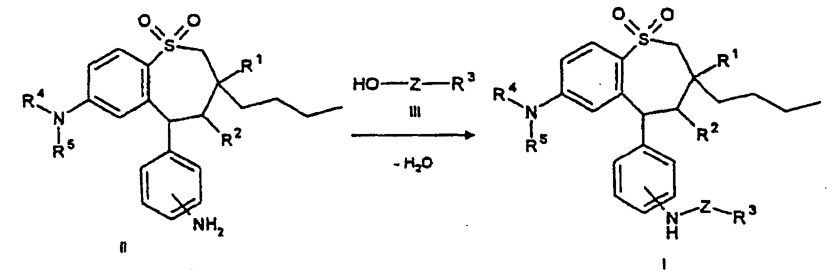 une amine de formule II, dans laquelle R¹, R², R⁴ et R⁵ possèdent les significations données à la formule I, à l'aide d'un composé de formule III dans laquelle R³ et Z possèdent l'une des significations données à la formule I, en éliminant l'eau, pour obtenir un composé de formule I, éventuellement on transforme le composé de formule 1 obtenu en un sel physiologiquement toléré ou un dérivé physiologiquement fonctionnel.

7. Médicament renfermant un ou plusieurs composés selon une ou plusieurs des revendications 1 à 5.

8. Médicament renfermant un ou plusieurs composés selon une ou plusieurs des revendications 1 à 5 et une ou plusieurs statines.

9. Composés selon une ou plusieurs des revendications 1 à 5 pour applications comme médicament pour le traitement des troubles de métabolisme de lipides.

10. Composés selon une ou plusieurs des revendications 1 à 5 pour applications comme médicament . pour le traitement ou la prévention de calculs biliaires.

11. Procédé pour la préparation d'un médicament renfermant un ou plusieurs composés selon une ou plusieurs des revendications 1 à 5, **caractérisé .en ce qu'**on mélange le principe actif avec un véhicule tolérés du point de vue pharmaceutique et le mélange est mis en forme d'administration appropriée.

12. Utilisation des composés selon une ou plusieurs des revendications 1 à 5, pour la préparation d'un médicament pour le traitement d'hyperlipidémie.

13. Utilisation des composés selon une ou plusieurs des revendications 1 à 5, pour la préparation d'un médicament agissant sur le niveau de cholestérol sérique.

14. Utilisation des composés selon une ou plusieurs des revendications 1 à 5, pour la préparation d'un médicament pour la prévention de phénomènes artériosclérotiques.

15. Utilisation des composés selon une ou plusieurs des revendications 1 à 5, pour la préparation d'un médicament pour le traitement ou la prévention de calculs biliaires.
